(19) Europäisches Patentamt
European Patent Office
Office européen des brevets

(11) Numéro de publication : **0 308 456 B1**

# (12) FASCICULE DE BREVET EUROPEEN

(45) Date de publication du fascicule du brevet : **04.12.91 Bulletin 91/49**

(51) Int. Cl.⁵ : **A61K 7/48,** A61K 7/06

(21) Numéro de dépôt : **88902868.4**

(22) Date de dépôt : **21.03.88**

(86) Numéro de dépôt international : **PCT/FR88/00147**

(87) Numéro de publication internationale : **WO 88/07363 06.10.88 Gazette 88/22**

## (54) COMPOSITION POUR LE TRAITEMENT DE L'EPIDERME.

(30) Priorité : **03.04.87 FR 8704737**

(43) Date de publication de la demande : **29.03.89 Bulletin 89/13**

(45) Mention de la délivrance du brevet : **04.12.91 Bulletin 91/49**

(84) Etats contractants désignés : **BE CH DE GB IT LI LU NL SE**

(56) Documents cités :
**FR-A- 2 164 801**
**Chemical Abstracts, vol. 87, no. 3, 18 juillet 1977 (Colombus, Ohio, US), B. Nunziata et al.: "Corneal radiofrequency burns: Effects of prostaglandins and 48/80", voir page 78, résumé no. 16342v, & Invest. Ophthalmol. visual sci. 1977, 16(4), 285-91**

(73) Titulaire : **SCHOEFF, Nadine**
**12, boulevard d'Andilly, "Les Atlantes"**
**F-95160 Montmorency (FR)**

(72) Inventeur : **POUJOL, Jean-Pierre**
**30 ter, rue Garibaldi**
**F-93400 Saint-Ouen (FR)**
Inventeur : **SCHOEFF, Nadine**
**12, bvd. d'Andilly "Les Atlantes"**
**F-95160 Montmorency (FR)**

(74) Mandataire : **Koch, Gustave et al**
**Cabinet PLASSERAUD 84, rue d'Amsterdam**
**F-75009 Paris (FR)**

EP 0 308 456 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne les compositions pour les soins locaux de l'épiderme.

Elle concerne plus précisément une composition pour le traitement du cuir chevelu, notamment pour le traitement des alopécies et de la pelade, comprenant au moins deux constituants, caractérisée en ce que l'un des cnstituants est un produit résultant de la condensation de la N-méthyl-p-méthoxyphénéthylamine avec le formaldéhyde, lequel produit de condensation est un produit de type polyaminé aromatique de formule générale $(C_{11}H_{15}NO)_n$, n représentant le plus souvent un nombre entier de 2 à 4.

Il semblerait que les produits de ce type résultent de l'enchaînement de motifs de formule

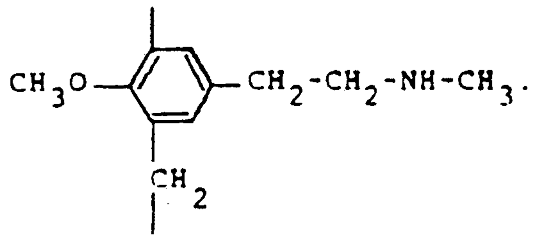

Un produit constitué apparemment par un mélange de di-, tri- et tétramères de ce motif est commercialisé par la Société SIGMA, BP 14508, Saint-Louis, MO 63178, FUA sous la dénomination "Compound 48/80" (Composé 48/80).

Ce produit ou "polyamine aromatique" se présente sous forme de chlorydrate de formule générale $(C_{11}H_{15}NO, HCl)_n$ de poids moléculaire (213,7)n.

Il s'agit d'une poudre blanche qui est soluble dans l'eau (50 mg/ml).

La "polyamine aromatique" est connue depuis les années 50 comme histamino-libérateur et est utilisée en allergologie comme produit de référence dans les essais cutanés.

Le produit commercialisé par la Société SIGMA sous la dénomination "Compound 48/80" a notamment déjà fait l'objet de tests concernant des brûleurs de la cornée (Chem.Abs.vol 87.1977.16342v). 2.

On a maintenant trouvé que de manière surprenante et inattendue les produits résultant de la condensation de la N-méthyl-p-méthoxyphénéthylamine avec le formaldéhyde tels que définis plus haut et appelés ci-après "produits de type polyamine aromatique" peuvent avantageusement être utilisés dans des compositions pour le traitement local de l'épiderme, notamment du cuir chevelu.

Plus précisément, on a trouvé que l'utilisation combinée des produits de type polyamine aromatique avec des principes réputés actifs au niveau de l'épiderme, accroît de façon considérable et inattendue l'activité de ces derniers, et ceci sans effets secondaires gênants et avec maintien d'une action localisée.

L'invention a donc de façon générale pour objet une composition pour le traitement local de l'épiderme comprenant au moins deux constituants actifs, caractérisée en ce que l'un de ces constituants est un produit résultant de la condensation de la N-méthyl-p-méthoxyphénéthylamine avec le formaldéhyde ou produit de type polyamine aromatique.

L'autre constituant actif est un composé ou mélange de composés connu pour être plus ou moins actif dans l'application envisagée.

Selon un mode de réalisation particulièrement avantageux et préféré, la composition selon l'invention est destinée au cuir chevelu, en particulier au traitement des alopécies et de la pelade.

Ainsi, selon un mode particulier de réalisation, l'invention a pour objet une composition pour le traitement du cuir chevelu, notamment pour le traitement des alopécies et de la pelade, caractérisée en ce qu'elle comprend une quantité efficace de composés protéiniques et une quantité non allergisante mais capable de provoquer la réponse des follicules pileux d'au moins un produit résultant de la condensation de la N-méthyl-p-méthoxyphénéthylamine avec le formaldéhyde.

Selon un mode préféré de réalisation, cette composition contient également des acides aminés soufrés.

Selon un mode avantageux de réalisation de l'invention, les composés protéiniques et éventuellement les acides aminés soufrés sont apportés sous forme d'extraits organiques d'origine humaine ou animale. Ils peuvent également être apportés sous forme d'un mélange de protéines et éventuellement d'acides aminés soufrés, obtenus par exemple par synthèse ou extraction et utilisés avantageusement dans les proportions observées dans les extraits organiques.

Un extrait organique convenant bien dans le cadre de la présente invention est par exemple le "complexe amino-protéique" lyophilisé provenant de broyat de tissu placentaire humain commercialisé sous la marque PHYLDERM POUDRE par les établissements Gattefossé, 36 chemin de Genas, 69804 Saint Priest (France).

Un autre extrait de ce type est celui commercialisé par la société Organotechnie, 27 avenue Jean Mermoz, 93120 La Courneuve (France).

De façon générale la composition selon l'invention peut se présenter sous toute forme convenant à l'application locale ou à l'administration par voie intradermique, en présence d'un excipient physiologiquement compatible.

Ainsi pour l'application locale, la composition selon l'invention peut se présenter sous forme de solution aqueuse, de préférence dans l'eau déminéralisée, ou sous forme de crème.

Pour l'application intradermique (ionisation), la composition selon l'invention peut se présenter sous forme d'une solution aqueuse dans l'eau déminéralisée, à un pH voisin de 7 et être conditionnée dans des ampoules, par exemple de 1 à 10 ml, notamment de 3 ml.

Pour l'administration par voie intradermique on utilise des ampoules du même type mais stérilisées.

Selon un mode préféré de réalisation, l'invention a pour objet une composition caractérisée en ce qu'elle est sous la forme d'une solution aqueuse ou lotion pour le traitement du cuir chevelu, notamment pour le traitement des alopécies et de la pelade, comprenant de $10^{-3}$ à 1 mg/ml de produit de type polyamine aromatique et de 0,1 à 10 mg/ml de mélange de composés protéiniques et d'acides aminés soufrés sous forme d'extrait placentaire lyophilisé.

Selon un autre mode préféré de réalisation, l'invention a pour objet une composition caractérisée en ce qu'elle est sous la forme d'une solution aqueuse pour l'application intradermique avec ionisation, dans le traitement du cuir chevelu, notamment dans le traitement des alopécies et de la pelade, conditionnée de préférence sous forme d'ampoules, comprenant de $10^{-3}$ à 1 mg/ml de produit de type polyamine aromatique et de 0,1 à 10 mg/ml de mélange de composés protéiniques et d'acides aminés soufrés sous forme d'extrait placentaire lyophilisé.

Selon un autre mode préféré de réalisation, l'invention a pour objet une composition caractérisée en ce qu'elle est sous la forme d'une solution aqueuse stérile pour l'administration intradermique dans le traitement du cuir chevelu, notamment dans le traitement des alopécies et de la pelade, conditionnée de préférence sous forme d'ampoules, comprenant de $10^{-3}$ à $10^{-1}$ mg/ml de produit de type polyamine aromatique et de 0,05 à 5 g/ml de mélange de composés protéiniques et d'acides aminés soufrés sous forme d'extrait placentaire lyophilisé.

Selon encore un autre mode préféré de réalisation, l'invention a pour objet une composition caractérisée en ce qu'elle est sous la forme d'une crème pour le traitement du cuir chevelu, notamment pour le traitement des alopécies et de la pelade, comprenant de $10^{-3}$ à 1 mg/g de produit de type polyamine aromatique et de 1 à 15 mg/g de mélange de composés protéiniques et d'acides aminés soufrés sous forme d'extrait placentaire lyophilisé.

L'excipient de la crème peut être constitué par tout excipient ou mélange d'excipients utilisé(s) dans les crèmes à usage pharmaceutique ou cosmétique et compatible(s) avec les principes actifs de la composition selon l'invention, notamment en présence d'un émulsifiant tel que celui commercialisé sous la marque Emulgade FS par la société Henkel.

La crème peut également comporter d'autres excipients tels que la cire d'abeille ou un polyalcool comme le glycérol.

Le pH des solution aqueuses est avantageusement ajusté au voisinage de 7 et le pH de la crème au voisinage de 5.

Pour abaisser le pH on peut utiliser tout acide pharmacologiquement acceptable et compatible avec les constituants de la solution ou de la crème, par exemple l'acide citrique.

Pour augmenter le pH on peut utiliser toute base pharmacologiquement acceptable et compatible avec les constituants de la solution ou de la crème, par exemple la triéthanolamine.

Tous ces produits, et notamment les crèmes, contiennent éventuellement un ou plusieurs agents de conservation tels que le méthylparabène.

Les exemples suivants sont destinés à mieux expliquer l'invention.

EXEMPLE 1

Lotion pour l'application locale sur le cuir chevelu.

100 l de solution contiennent :

- produit de type polyamine aromatique commercialisé par la société SIGMA sous la dénomination "Compound 48/80" : 10 g
- "complexe amino-protéique" lyophilisé provenant de broyat de tissu placentaire humain, en poudre, commercialisé sous la marque PHYLDERM par la Société Gattefossé : 400 g

- méthylparabène : 3l
- éventuellement acide citrique ou triéthanolamine qsp pH au voisinage de 7
- eau déminéralisée : qsp 100 l.

EXEMPLE 2

Ampoules pour l'application intradermique sur le cuir chevelu avec ionisation

100 ml de solution contiennent :
- produit de type polyamine aromatique commercialisé par la Société SIGMA sous la dénomination "Compound 48/80" : 10 mg
- "complexe amino-protéique" lyophilisé provenant de broyat de tissu placentaire humain, en poudre, commercialisé sous la marque PHYLDERM par la Société Gattefossé : 400 mg
- méthylparabène : 3 ml
- éventuellement acide citrique ou triéthanolamine qsp pH au voisinage de 7.

La solution est conditionnée dans des ampoules en plastique de 3 ml avec tube applicateur.

EXEMPLE 3

Ampoules pour l'administration intradermique dans le cuir chevelu :

100 ml de solution contiennent :
- produit de type polyamine aromatique commercialisé par la Société SIGMA sous la dénomination "Compound 48/80" : 1 mg
- "complexe amino-protéique" lyophilisé provenant de broyat de tissu placentaire humain, en poudre, commercialisé sous la marque PHYLDERM par la Société Gattefossé : 100 g
- éventuellement acide citrique ou triéthanolamine qsp pH au voisinage de 7.

La solution est conditionnée dans des ampoules en verre de 3 ml qui sont fermées et le tout est stérilisé.

EXEMPLE 4

Crème pour l'application sur le cuir chevelu :

100 g de crème contiennent :

| | |
|---|---|
| - Compound 48/80 de la société SIGMA : | 10 mg |
| - PHYLDERM POUDRE de la société Gattefossé : | 800 mg |
| - Acide stéarique : | 4 g |
| - Alcool cétylique : | 4 g |
| - Emulgade $^{R}$ FS : | 8 g |
| - Lanoline : | 6 g |
| - Cire d'abeille : | 15 g |
| - Huile de vaseline : | 20 g |
| - Eau déminéralisée : | qsp 100 g |

On dissout les principes actifs (Compound 48/80 et PHYLDERM) dans l'eau déminéralisée. Par ailleurs, on mélange les autres constituants de la crème, on chauffe le mélange et on laisse refroidir sous agitation lente jusqu'à 25°C. On incorpore ensuite la phase aqueuse préparée séparément et, si nécessaire, on ajuste le pH comme indiqué plus haut.

Applications des compositions selon l'invention.

EXEMPLE 5

Application de la lotion de l'exemple 1.

Cette lotion peut être utilisée, en particulier pour le traitement de l'alopécie, en application locale.

La dose journalière est d'environ 50 gouttes à répartir sur la zone alopécique.

EXEMPLE 6

Traitement par ionisation

On utilise avantageusement les ampoules de l'exemple 2 que l'on fait pénétrer dans le cuir chevelu, sous ionisation, en 20 minutes. Ce traitement se fait à raison de 1 à 2 fois par semaine en augmentant progressivement la dose toutes les 2 à 3 semaines, à savoir selon le schéma 1 ml, 1,5 ml, 2 ml, 2,5 ml, 3 ml.

EXEMPLE 7

Utilisation de la crème de l'exemple 4 pour la puvathérapie.

L'application se fait en couche fine 10 minutes après la fin de l'ionisation.

La couche est d'autant plus fine que les séances sont plus rapprochées, mais son épaisseur dépend essentiellement de la réactivité cutanée du sujet.

Dans le cas de la pelade, cette application a lieu en générale 2 fois par semaine, alors que dans le cas de l'alopécie une application par semaine suffit en général.

EXEMPLE 8

Application par voie intradermique.

On utilise les ampoules injectables de l'exemple 3.

La détermination de la dose du médiateur chimique est fonction de la réactivité cutanée et de l'âge du sujet. On a observé que quelle que soit la dose utilisée, la surface d'oedème réactionnel diminue quand l'âge augmente.

On a ainsi constaté de façon statistique que la réactivité est :

- maximale de 10 à 29 ans,
- intermédiaire de 30 à 49 ans,
- minimale au-dessus de 50 ans.

Une étude globale effectuée sur 70 sujets a montré que c'est en général à la dilution de $10^{-2}$ mg/ml que pour le pourcentage réactionnel est le plus grand.

EXPERIMENTATIONS CLINIQUES ET PARA-CLINIQUES

I. Traitement de l'alopécie

L'association polyamine aromatique plus complexe protéinique des exemples 1 et 2 a été expérimentée sur 2000 patients présentant une alopécie andro-génétique.

Il s'agissait de sujets de sexe masculin présentant une alopécie androgénétique de types 3 et 4 du tableau de Hamilton. Dans chaque cas, la composition a été appliquée de la façon décrite précédemment.

La réaction cutanée s'est avérée excellente dans 95 % des cas et a permis la conduite d'un traitement régulier dans 100 % des cas.

Résultats

1. Action sur la chute des cheveux

- après un mois de traitement : diminution de la chute dans 55 % des cas;

– après deux mois de traitement : arrêt de la chute des cheveux dans 75 % des cas ;
– après trois mois de traitement : arrêt de la chute des cheveux dans 85 % des cas.

2. Repousse

Elle se contrôle à partir de 4è mois, elle n'est obtenue que sur des follicules pileux ayant des cellules germinatives encore vivantes. Elle est peu fréquente dans les golfes temporaux, les cellules germinatives y étant rarement vivantes dans les types 3 et 4 de calvitie du tableau de Hamilton.

La repousse reste possible chez le sujet féminin dans 80 % des cas.

La calvitie se comble à partir de son pourtour, les derniers cheveux tombés étant les premiers remplacés.

Dans 70 % des cas, on observe des cheveux de repousse sur l'ensemble de la calvitie après 6 à 7 mois de traitement.

L'examen para-clinique (étude par tricogramme) permet de corroborer la tendance favorable constatée par l'examen clinique.

Les appréciations sont faites au jour 0 et au jour 6 mois, par comptage du nombre de cheveux dans différentes phases (anagène, catagène, télogène, dystrophie I et dystrophie II).

Sur la base de ces critères on constate que l'on obtient :
– dans 55 % des cas une normalisation de la formule pilaire ;
– dans 35 % des cas une amélioration sans normalisation de la formule pilaire ; et
– dans 10 % des cas, aucune modification de la formule pilaire.

I. Traitement de la pelade

Dans le cas de pelade, on utilise la composition de l'exemple 4, en application locale, sur la zone peladique.

On obtient un résultat satisfaisant (repousse des cheveux) sur les pelades localisées, dans 100 % des cas, après 6 à 12 mois de traitement (quel que soit le nombre de pelades et leur localisation).

Dans le cas des pelades déclavantes, les expérimentations en cours donnent des résultats qui paraissent satisfaisants.

III. Expérimentation clinique relative à la tenue des cheveux de repousse

La composition pharmaceutique selon l'invention, notamment la composition des exemples 1 à 4, permet la pousse du cheveu à partir d'une cellule germinative vivante incapable d'assurer seule sa fonction. La composition selon l'invention rétablit cette fonction grâce à l'action conjuguée de ses composants.

L'expérimentation clinique sur sept années a permis de constater un maintien de l'acquis dans 80 % des cas avec un traitement d'entretien de 1 à 2 cures annuelles.

Le tableau suivant montre les résultats d'une étude comparative entre les résultats obtenus sur une période de six mois avec une solution de la polyamine aromatique seule, une solution du complexe protéinique seul et la composition selon l'invention (lotion de l'exemple 1).

## TABLEAU

ACTION SUR LA REPOUSSE

| | Pas de modification | Début de repousse sous forme de duvet et de petits cheveux fins | Repousse nette + diminution du diamètre des zones alopéciques | Repousse très importante et très nette diminution des zones alopéciques |
|---|---|---|---|---|
| polyamine aromatique seule | 80 % | 20 % ne tiennent pas | 0 | 0 |
| complexe protéinique seul | 40 % | 60 % ne tiennent pas | 0 | 0 |
| complexe protéinique + polyamine aromatique | 5 % | 7 % | 38 % | 50 % |

A l'étude de ce tableau on constate que la polyamine utilisée seule permet un léger début de repousse des cheveux sous forme de duvet et de petits cheveux fins, le complexe protéinique permet un début de repousse amélioré mais sans repousse nette ni repousse importante alors que la composition selon l'invention permet d'obtenir de très bons résultats, à savoir notamment une repousse très importante et une très nette diminution des zones alopéciques pour 50 % des sujets, alors que la repousse est nette dans 38 % des cas.

Il convient de noter qu'en règle générale la composition selon l'invention produit ses effets sur le lieu d'application, sans essaimage en dehors de ce lieu. La composition selon l'invention a donc une activité strictement localisée au lieu d'application que ce soit par voie locale ou sous-cutanée.

ETUDE DE LA TOXICITE

La composition selon l'invention ne provoque pas de dommage tissulaire et a une action uniforme en application sur un organe isolé.

L'évaluation de l'irritation primaire cutanée et de l'irritation oculaire chez le lapin, et le test d'inocuité par voie orale chez le rat ont été réalisés conformément aux méthodes publiées au Journal Officiel de la République Française en date des 21 février 1982 et 21 septembre 1984, en accord avec les recommandations de bonnes pratiques de laboratoires (France, Ministère de la Santé - Instructions 1065 du 31 mai 1983).

Les études ont été réalisées au centre international de toxicité à Meserey 27005 Evreux - France.

Modalités expérimentales

Irritation primaire cutanée chez le lapin :

Elle a été évaluée après application topique de 0,5 ml de la lotion de l'exemple 1 sur la peau scarifiée et non scarifiée de 6 lapins mâles néo-zélandais (animaux reçus du centre d'élevage de l'Abbaye de Belle-fontaine 49122 Begrolles en Mauges - France) d'un poids moyen de 2,5 kg.

Le flan droit des animaux a été scarifié (trois scarifications parallèles sur une longueur d'environ 2,5 cm, espacées de 0,5 cm) les scarifications de l'épiderme ont été réalisées à l'aide d'un vaccinostyle, sans atteindre le derme.

0,25 ml de la composition selon l'invention a été directement appliqué sur une surface de 6 $cm^2$ puis recouvert d'un carré de gaze hydrophile Codex. Les animaux portaient un collier autour du cou pour les empêcher de se lécher.

Le produit et les carrés de gaze ont été maintenus en contact avec la peau au moyen d'un pansement occlusif.

24 heures après l'application, les pansements ont été enlevés. La période d'observation des animaux a été de 3 jours.

On n'a pas observé d'érythème. Le produit est donc considéré comme non irritant.

Irritation oculaire chez le lapin

Cette irritation a été évaluée après instillation de 0,1 ml de la lotion de l'exemple 1 dans le cul de sac conjonctival de 6 lapins mâles néo-zélandais de 2,5 kg.

Modalités

0,1 ml de la lotion selon l'invention est instillé dans le cul de sac conjonctival inférieur gauche de chaque lapin, à l'aide d'une seringue. Les paupières sont maintenues en contact quelques secondes afin d'éviter toute perte de substance. L'oeil droit ne reçoit aucun produit et sert de témoin. Pour le traitement et pendant une heure, les animaux sont placés en boîte à contension en matière plastique. L'instillation est suivie d'une période d'observation des animaux de 7 jours.

Aucune réaction oculaire n'est observée.

Test d'innocuité par voie orale chez le rat

Ce test a été effectué selon la norme OCDE n°401 concernant l'étude de la toxicité aiguë des produits chimiques par voie orale.

Pour l'étude on a utilisé des rats Sprague-Dawley : CD (SD)BR qui est l'espèce recommandée pour ce type d'expérimentation. Ces rats provenaient de l'élevage Charles River France (76410 Saint-Aubin lès Elboeuf - France).

Leur poids au début de l'expérimentation était de 110 à 160 g.

Un volume unique de 5 ml par kg de la lotion de l'exemple 1 été administré à l'aide d'une canule de gavage à chaque animal d'un groupe de dix (5 mâles et 5 femelles). L'observation des signes cliniques a été effectuée au moins une fois par jour pendant les 14 jours suivants.

Aucune mortalité ni aucun signe de toxicité n'a été enregistré tout au long de l'étude.

L'administration par voie orale de la composition de l'exemple 1 selon l'invention n'entraîne donc aucun signe de toxicité à la dose de 5 ml par kg.

## Revendications

1. Composition pour le traitement du cuir chevelu, notamment pour le traitement des alopécies et de la pelade, comprenant au moins deux constituants, caractérisée en ce que le premier de ces constituants est un produit résultant de la condensation de la N-méthyl-p-méthoxyphénéthylamine avec le formaldéhyde, c'est-à-dire un produit de type polyamine aromatique de formule générale $(C_{11}H_{15}NO)_n$, n représentant un nombre entier de 2 à 4, et que le deuxième constituant est un mélange de composés protéiniques.

2. Composition selon la revendication 1, caractérisée en ce que le produit de type polyamine aromatique est mis en oeuvre sous la forme de son chlorhydrate dont la formule générale est $(C_{11}H_{15}NO,HCl)_n$.

3. Composition selon la revendication 1 ou 2, caractérisée en ce qu'elle comprend des acides aminés soufrés.

4. Composition selon l'une des revendications 1 à 3, caractérisée en ce que les composés protéiniques et les acides aminés soufrés éventuels sont apportés sous forme d'extraits organiques.

5. Composition selon l'une des revendications 1 à 4, caractérisée en ce que les composés protéiniques et les acides aminés soufrés éventuels ont été obtenus par synthèse ou extraction et sont utilisés en mélange de préférence dans les proportions observées dans les extraits organiques.

6. Composition selon l'une des revendications 2 à 5, caractérisée en ce qu'elle est sous la forme d'une solution aqueuse ou lotion comprenant de $10^{-3}$ à 1 mg/ml de produit de type polyamine aromatique et de 0,1 à 10 mg/ml de mélange de composés protéiniques et d'acides aminés soufrés sous forme d'extrait placentaire lyophilisé.

7. Composition selon l'une des revendications 2 à 5, caractérisée en ce qu'elle est sous la forme d'une solution aqueuse pour l'application intradermique avec ionisation, conditionnée de préférence sous forme

d'ampoules et comprenant de $10^{-3}$ à 1 mg/ml de produit de type polyamine aromatique et de 0,1 à 10 mg/ml de mélange de composés protéiniques et d'acides aminés soufrés sous forme d'extrait placentaire lyophilisé.

8. Composition selon l'une des revendications 2 à 5, caractérisée en ce qu'elle est sous la forme d'une solution aqueuse stérile pour l'administration intradermique, conditionnée de préférence sous forme d'ampoules et comprenant de $10^{-3}$ à $10^{-1}$ mg/ml de produit de type polyamine aromatique et de 0,05 à 5 g/ml de mélange de composés protéiniques et d'acides aminés soufrés sous forme d'extrait placentaire lyophilisé.

9. Composition selon l'une des revendications 2 à 5, caractérisée en ce qu'elle est sous la forme d'une crème comprenant de $10^{-3}$ à 1 mg/g de produit de type polyamine aromatique et de 1 à 15 mg/g de mélange de composés protéiniques et d'acides aminés soufrés sous forme d'extrait placentaire lyophilisé.

10. Procédé de traitement local de l'épiderme, notamment du cuir chevelu, caractérisé en ce qu'il comprend l'utilisation d'au moins une composition sous la forme d'une solution ou d'une crème, selon l'une des revendications 1 à 9.

## Patentansprüche

1. Zusammensetzung für die Behandlung der Kopfhaut, insbesondere für die Behandlung von jeglicher Form von Haarausfall, wobei die Zusammensetzung wenigstens zwei Bestandteile aufweist,
**dadurch gekennzeichnet**,
daß der erste Bestandteil ein Produkt ist, das aus der Kondensation des N-Methyl-p-Methoxyphenethylamin mit Formaldehyd resultiert, d.h. das ein Produkt vom Typ eines aromatischen Polyamins der allgemeinen Formel $(C_{11}H_{15}NO)_n$ ist, wobei n eine ganze Zahl zwischen 2 und 4 ist, und daß der zweite Bestandteil eine Mischung proteinischer Komponenten ist.

2. Zusammensetzung nach Anspruch 1,
**dadurch gekennzeichnet**,
daß das Produkt vom Typ aromatischer Polyamine in Form seines Chlorhydrates, dessen allgemeine Formel $(C_{11}H_{15}NO,HCl)_n$ ist, hergestellt ist.

3. Zusammensetzung nach Anspruch 1 oder 2,
**dadurch gekennzeichnet**,
daß sie schwefelhaltige Aminosäuren aufweist.

4. Zusammensetzung nach einem der Ansprüche 1 bis 3,
**dadurch gekennzeichnet**,
daß die proteinischen Komponenten und die schwefelhaltigen Aminosäuren in Form von organischen Extrakten zugegeben werden.

5. Zusammensetzung nach einem der Ansprüche 1 bis 4,
**dadurch gekennzeichnet**,
daß die proteinischen Komponenten und die eventuellen schwefelhaltigen Aminosäuren durch Synthese oder Extraktion erhalten worden sind und daß sie gemischt vorzugsweise in Proportionen verwendet werden, die in den organischen Extrakten beobachtet worden sind.

6. Zusammensetzung nach einem der Ansprüche 2 bis 5,
**dadurch gekennzeichnet**,
daß sie in Form einer wässrigen Lösung oder einer Milch mit $10^{-3}$ bis 1 mg/ml des Produktes vom Typ aromatischer Polyamine und mit 0,1 bis 10 mg/ml der Mischung proteinischer Komponenten und schwefelhaltiger Aminosäuren in Form von lyophilisiertem plazentärem Extrakt vorliegt.

7. Zusammensetzung nach einem der Ansprüche 2 bis 5,
**dadurch gekennzeichnet**,
daß sie in Form einer wässrigen Lösung zur intradermischen Anwendung mit Ionisation vorliegt, wobei sie vorzugsweise in Ampullen abgefüllt ist, und $10^{-3}$ bis 1 mg/ml des Produktes vom Typ aromatischer Polyamine und 0,1 bis 10 mg/ml der Mischung proteinischer Komponenten und schwefelhaltiger Aminosäuren in Form von lyophilisiertem plazentärem Extrakt aufweist.

8. Zusammensetzung nach einem der Ansprüche 2 bis 5,
**dadurch gekennzeichnet**,
daß sie in Form einer sterilen wässrigen Lösung zur intradermischen Anwendung vorliegt, wobei sie vorzugsweise in Ampullen abgefüllt ist, und $10^{-3}$ bis $10^{-1}$ mg/ml des Produktes vom Typ aromatischer Polyamine und 0,05 bis 5 mg/ml der Mischung proteinischer Komponenten und schwefelhaltiger Aminosäuren in Form von lyophilisiertem plazentärem Extrakt aufweist.

9. Zusammensetzung nach einem der Ansprüche 2 bis 5,
**dadurch gekennzeichnet**,

daß sie in Form einer Creme vorliegt, und $10^{-3}$ bis 1 mg/g des Produktes vom Typ aromatischer Polyamine und 1 bis 15 mg/g der Mischung proteinischer Komponenten und schwefelhaltiger Aminosäuren in Form von lyophilisiertem plazentärem Extrakt aufweist.

10. Verfahren zur lokalen Behandlung der Epidermis, insbesondere der Kopfhaut,
**dadurch gekennzeichnet**,
daß es die Verwendung wenigstens einer Zusammensetzung in Form einer Lösung oder einer Creme gemäß einem der Patentansprüche 1 bis 9 aufweist.

## Claims

1. Composition for the treatment of the scalp, especially for treating alopecia and pelada, comprising at least two constituents, **characterized in that** the first one of these constituents is a product resulting from condensation of N-methyl-methoxyphenethylamine with formaldehyde, that means a product of the aromatic polyamine-type of the general formular $(C_{11}H_{15}NO)_n$, wherein n represents an integer between 2 and 4, and in that the second constituent is a mixture of proteinic compounds.

2. Composition according to claim 2, **characterized in that** the product of the aromatic polyamine-type is obtained under the form of its hydrochloride of the general formula $(C_{11}H_{15}NO,HCl)_n$.

3. Composition according to claim 1 or 2, **characterized in that** it comprises sulfurized amino acids.

4. Composition according to one of claims 1 through 3, **characterized in that** the proteinic compounds and eventual sulfurized amino acids are introduced under the form of organic extracts.

5. Composition according to one of claims 1 through 4, **characterized in that** the proteinic compounds and the eventual sulfurized amino acids are obtained by synthesis or by extraction and used as a mixture preferably in the proportions observed in the organic extracts.

6. Composition according to one of the claims 2 through 5, **characterized in that** it has the form of an aqueous solution or lotion comprising $10^{-3}$ to 1 mg/ml of product of the aromatic polyamine-type and 0.1 to 10 mg/ml of mixture of proteinic compounds and of sulfurized amino acids under the form of lyophilized placental extract.

7. Composition according to one of claims 2 through 5, **characterized in that** it has the form of an aqueous solution for intradermic application with ionization, preferably conditioned under the form of ampullae and comprising $10^{-3}$ to 1 mg/ml of product of the aromatic polyamine-type and 0.1 to 10 mg/ml of mixture of proteinic compounds and of sulfurized amino acids under the form of lyophilized placental extract.

8. Composition according to one of claims 2 through 5, **characterized in that** it has the form of a sterile aqueous solution for intradermic administration, preferably conditioned under the form of ampullae and comprising $10^{-3}$ to $10^{-1}$ mg/ml of product of the aromatic polyamine-type and 0.05 to 5 mg/ml of mixture of proteinic compounds and sulfurized amino acids under the form of lyophilized placental extract.

9. Composition according to one of claims 2 through 5, **characterized in that** it has the form of a cream comprising $10^{-3}$ to 1 mg/g of product of the aromatic polyamine-type and 1 to 15 mg/g of mixture of proteinic compounds and sulfurized amino acids under the form of lyophilized placental extract.

10. Method of local treatment of the epiderme, especially of the scalp, **characterized in that** it comprises the utilization of at least one composition under the form of a solution or a cream according to one of claims 1 through 9.